(19) Europäisches Patentamt
European Patent Office
Office européen des brevets

(11) **EP 2 437 192 A1**

(12) **EUROPEAN PATENT APPLICATION**

(43) Date of publication:
**04.04.2012 Bulletin 2012/14**

(51) Int Cl.:
***G06F 19/24*** *(2011.01)* ***C12Q 1/68*** *(2006.01)*

(21) Application number: **10177129.3**

(22) Date of filing: **16.09.2010**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**
Designated Extension States:
**BA ME RS**

(71) Applicants:
- **Roche Diagnostics GmbH**
  **68305 Mannheim (DE)**
  Designated Contracting States:
  **DE**
- **F. Hoffmann-La Roche AG**
  **4070 Basel (CH)**
  Designated Contracting States:
  **AL AT BE BG CH CY CZ DK EE ES FI FR GB GR HR HU IE IS IT LI LT LU LV MC MK MT NL NO PL PT RO SE SI SK SM TR**

(72) Inventors:
- **Bechler, Ingrid**
  **82538 Geretsried (DE)**
- **Sagner, Gregor**
  **82377 Penzberg (DE)**

(74) Representative: **Burger, Alexander**
**Roche Diagnostics GmbH**
**Patent Department (TR-E)**
**Postfach 11 52**
**82372 Penzberg (DE)**

(54) **Relative quantification analysis of multi-parametric PCR experiments**

(57) An analysis method for quantitative PCR experiments comprising multiple genes of interest, multiple samples and multiple conditions is provided. It was found that for calculating the relative expression status of multiple target genes from multiple samples under multiple different conditions, a calculation method based on up to three different parameters has to be performed. In addition to normalization against one or multiple reference gene(s) and normalization against a reference sample (calibrator), a third normalization step against a base value has to be performed in a target-, sample- and condition specific manner.

EP 2 437 192 A1

## Description

### Background of the Invention

One of the major applications of real time PCR systems is relative quantification of gene expression levels and gene copy number. Relative quantification avoids the need of measuring absolute quantities of mRNA or gene copies and thus avoids the use of standard dilutions with known copy numbers.

Instead, by normalization of the gene of interest (target gene) against one or multiple unregulated reference gene(s) (often called housekeeping genes) from the same sample material, a relative target/reference ratio is calculated. This calculation might be based on PCR efficiencies derived from standard dilution series with unknown absolute copy numbers. This relative ratio normalizes against sample-to-sample differences caused by different sample amount, sample quality and differences during the purification process.

In addition to the relative target/reference ratio calculation, a second normalization step can be performed. Hereby the target/reference ratio of each sample is divided by the target/reference ratio of a special sample often called calibrator, resulting in normalized ratios or normalized expression. This step normalizes against experiment run differences and different sensitivities for detecting target and reference genes, e.g. caused by probe and label amount and quality, probe annealing efficiency and probe hydrolysis efficiency.

This calculation of normalized ratios or normalized expression is implemented in real time PCR analysis software of multiple suppliers (e.g. Roche LightCycler 480 system software, Applied Biosystems StepOne Plus / 7500 / 7900HT system software, Biorad CFX Manager software, Biogazelle qBase Plus software etc.).

In recent years the experiments to answer scientific questions became more complex and relative quantification analyses are usually performed with multiple target genes from multiple samples. Furthermore, it is frequently required to measure the gene expression status or gene copy status of each sample and each gene at different time points or under different conditions (e.g. after treatment with different substances or in different development phases). For each of these measurements it might be relevant to calculate normalized ratios, especially when the data collection encompasses multiple experiment runs (multiple separate microwell plates).

To generate a meaningful result it is necessary to refer the different measurements to a common basis (e.g. an untreated state or an early development phase). This has to be done in a target-specific and sample-specific manner.

None of the currently available analysis methods provides strategies for this kind of analysis. In case that multiple genes of interest, multiple samples and multiple conditions are used in a relative quantification experiment, current analysis methods require to fragment the experiment in multiple separate analyses each containing only one gene or one sample.

Especially in relative quantification analyses encompassing multiple experiments, one experiment is frequently used as calibrator plate. All additional experiments are normalized against this calibrator plate. This calculation is only possible when just one sample or one target gene is contained in the relative quantification analysis, as only two parameters are usable for normalization.

The independent inter-run calibrator provided in certain real time PCR software solutions is not used in the ratio or normalized ratio calculation process and thus does not allow compensating for gene-specific and sample-specific run-to-run differences.

### Summary of the Invention

It is the task of the present invention to provide an analysis method for experiments comprising multiple genes of interest, multiple samples and multiple conditions. It was found that for calculating the relative expression status of multiple target genes from multiple samples under multiple different conditions, a calculation method based on up to three different parameters has to be performed. In addition to normalization against one or multiple reference gene(s) and normalization against a reference sample (calibrator), a third normalization step against a base value has to be performed in order to perform a target-specific, sample-specific and condition-specific analysis.

One aspect of the present invention is a method for the analysis of PCR experiments with a plurality of experimental conditions and a plurality of samples each comprising one or more target nucleic acids and one or more reference nucleic acids, said method comprising

a) a plurality of PCR amplifications of target nucleic acids and reference nucleic acids,
b) a first normalization step based on referring each target nucleic acid amplification to the amplification of one or more reference nucleic acids to obtain relative target/reference ratios,
c) a second normalization step based on referring the relative target/reference ratios calculated in step b) to a relative target/reference ratio obtained at an experimental base condition to obtain scaled target/reference ratios, and
d) an analysis of the PCR experiment based on comparing the scaled target/reference ratios calculated in step c)

in a target-, sample- and condition specific manner.

Throughout the present invention the expression "experimental condition" summarizes all parameters that can have an influence on gene expression measured by the PCR amplification, e.g. a point in time to monitor the expression during a certain period or a set of compounds to screen their influence on gene expression. The "experimental base condition" is the initial condition within a series of experiments, whereas the "base value" is the result of a PCR amplification performed at this experimental base condition.

Analogous to measuring the gene expression status under different conditions, the gene copy status can also be determined under different conditions.

Within this aspect of the present invention the first normalization is based on the first parameter, namely the nucleic acid (or gene type). The PCR amplification of target nucleic acids is normalized using the PCR amplification of reference nucleic acids to provide relative target/reference ratios (RRs). Said reference nucleic acids are called housekeeping genes in gene expression determination. In gene copy determination, e.g. single copy genes can be used as reference nucleic acids. It is possible to perform this normalization based on one or more of these reference genes, e.g. using the arithmetic mean value of the amplification result (Cq value) of several reference genes.

The second normalization is based on the second parameter, namely the experimental condition. Here, the PCR amplification result of target nucleic acids is normalized in a sample-specific manner using the PCR amplification result of target nucleic acids obtained at a certain experimental condition called the "experimental base condition" to provide a scaled ratio. When the second normalization step is performed in addition to the first normalization step, scaled target/reference ratios (SRs) are obtained.

## Detailed Description of the Invention

One aspect of the present invention is a method for the analysis of PCR experiments with a plurality of experimental conditions and a plurality of samples each comprising one or more target nucleic acids and one or more reference nucleic acids, said method comprising

a) a plurality of PCR amplifications of target nucleic acids and reference nucleic acids,
b) a first normalization step based on referring each target nucleic acid amplification to the amplification of one or more reference nucleic acids to obtain relative target/reference ratios,
c) a second normalization step based on referring the relative target/reference ratios calculated in step b) to a relative target/reference ratio obtained at an experimental base condition to obtain scaled target/reference ratios, and
d) an analysis of the PCR experiment based on comparing the scaled target/reference ratios calculated in step c) in a target-, sample- and condition specific manner.

If an analysis is performed over multiple experimental runs and each experimental run monitors one or multiple experimental conditions without running the base condition in every experimental run (see example 3), a third normalization step using a third parameter is necessary. This third parameter is the sample type. The sample type allows to define a so called calibrator sample. All samples of type "unknown" are normalized against the run-specific calibrator. This normalization step allows to correct run-to-run differences in a gene-specific manner when the base condition is not used in every experimental run. If the third normalization step is performed in addition to the first and second normalization step, scaled and normalized target/reference ratios (SNRs) are obtained.

With respect to the succession of the different normalization steps at least two options exist, because from a mathematical point of view the result of the successive ratios will be the same.

A preferred method according to the present invention is a method further comprising a third normalization step based on referring the scaled target/reference ratios calculated in step c) to a scaled target/reference ratio from a calibrator sample to obtain scaled and normalized target/reference ratios, and wherein said analysis of the PCR experiment in step d) is based on comparing said scaled and normalized target/reference ratios in a target-, sample- and condition specific manner.

In this alternative with three normalization steps, the target nucleic acid amplification is first normalized with respect to a reference nucleic acid, then scaled and finally normalized with respect to a calibrator sample.

Another preferred method according to the present invention is a method further comprising a third normalization step based on referring the relative target/reference ratios calculated in step b) to a relative target/reference ratio from a calibrator sample to obtain normalized target/reference ratios, wherein the normalization in step c) is based on referring the normalized target/reference ratios to a normalized target/reference ratio obtained at an experimental base condition to obtain scaled and normalized target/reference ratios, and wherein said analysis of the PCR experiment in step d) is based on comparing said scaled and normalized target/reference ratios in a target-, sample- and condition specific manner.

In this alternative with three normalization steps, the target nucleic acid amplification is first normalized with respect to a reference nucleic acid, then normalized with respect to a calibrator sample and finally scaled.

When all three parameters and normalization steps are provided, the full setup and workflow flexibility for relative quantification experiments is enabled and results can correctly be sorted by all variables occurring in experimental design.

As mentioned before, the expression "experimental condition" summarizes all parameters that can have an influence on the gene expression or gene copy number, e.g. a point in time to monitor the amplification during a certain period or a set of compounds to screen their influence on gene expression or copy number.

Yet another preferred method according to the present invention is a method, wherein said experimental conditions are a point in time or a treatment with a certain compound.

With respect to the setup of an experiment the method of the present invention is not restricted. The PCR amplifications can be performed in a plurality of single vessels or in one or more arrangements of vessels such as in microwell plates or well stripes.

A preferred method according to the present invention is a method, wherein all PCR amplifications are performed within wells of one microwell plate.

Another preferred method according to the present invention is a method, wherein all PCR amplifications are performed within wells of multiple microwell plates.

The person skilled in the art will know about the different formats of such microwell plates and it is preferred to use plates that fulfill the standard dimensions such that the commercially available handling devices can be used.

A more preferred method according to the present invention is a method, wherein said microwell plates are 96 well plates, 384 well plates or 1536 well plates.

A preferred method according to the present invention is a method, wherein the relative target/reference ratios of a sample ($RR_s$) are calculated according to

$$RR_s = E_R^{Cq\,(Rs)} / E_T^{Cq(Ts)}\ ,$$

with:

- $E_R$ = PCR efficiency of a reference nucleic acid,
- $E_T$ = PCR efficiency of a target nucleic acid and
- $C_q$ = cycle of quantification for a reference nucleic acid in a sample (Rs) and for a target nucleic acid in the same sample (Ts).

The theoretical value of the PCR efficiency is 2 resulting in doubling the amount of nucleic acids in each PCR cycle. In real life, the PCR efficiency can differ between e.g. the nucleic acid and the reference nucleic acid. Consequently, it is preferred to treat the PCR efficiency as a variable and ways to handle this variances are e.g. described in EP 1138783B1 or US 7,125,691.

Another preferred method according to the present invention is a method, wherein the scaled target/reference ratios (SR) are calculated according to

$$SR = RR_S / RR_B\,,$$

with:

- $RR_B$ is the relative target/reference ratio in the sample measured at the experimental base condition (B).

In this normalization step scaled ratios (SR) are calculated by dividing all relative ratios by the relative ratio of the selected base value. Therefore, SR is calculated according to

$$SR = RR_S / RR_B = (E_R^{Cq(Rs)} / E_T^{Cq(Ts)}) \,/\, (E_R^{Cq(Rs_{,B})} / E_T^{Cq(Ts_{,B})}),$$

with

Cq (Rs) or Cq ($R_{S,B}$) being the cycle of quantification for a reference nucleic acid in the sample at a certain condition or in the same sample at the experimental "base" condition and Cq (Ts) or Cq ($T_{S,B}$) for a target nucleic acid in the sample at a certain condition or in the same sample at the experimental "base" condition.

Yet another preferred method according to the present invention is a method, wherein the normalized target/reference ratio (NR) are calculated according to

$$NR = RR_S / RR_C ,$$

with:

$RR_S$ is the relative ratio in the sample and
$RR_C$ is the relative target/reference ratio in the calibrator sample.

Therefore, normalized Ratios (NR) are calculated according to

$$NR = RR_S / RR_C = (E_R^{Cq(Rs)}/E_T^{Cq(Ts)}) / (E_R^{Cq(Rc)}/E_T^{Cq(Tc)}) ,$$

with

Cq (Rs) or Cq (Rc) being the cycle of quantification for a reference nucleic acid in the sample or the calibrator sample and Cq (Ts) or Cq (Tc) for a target nucleic acid in the sample or the calibrator sample.

Still another preferred method according to the present invention is a method, wherein the scaled and normalized ratios (SNR) are calculated according to

$$SNR = NR_S / NR_B ,$$

with:

$NR_S$ being the normalized target/reference ratios of a sample at a certain experimental condition and $NR_B$ the normalized target/reference ratio at the experimental base condition (B).

Therefore, scaled and normalized ratios (SNR) are calculated according to

$$SNR = NR_S / NR_B = \frac{(E_R^{Cq(Rs)}/E_T^{Cq(Ts)}) / (E_R^{Cq(Rc)}/E_T^{Cq(Tc)})}{(E_R^{Cq(Rs,_B)}/E_T^{Cq(Ts,_B)}) / (E_R^{Cq(Rc,_B)}/E_T^{Cq(Tc,_B)})}$$

Yet another preferred method according to the present invention is a method, wherein the PCR amplification in each well is a mono color amplification.
Still another preferred method according to the present invention is a method, wherein the PCR amplification in each well is a multi color amplifications, preferably a dual color amplification.

**Example 1 : Relative quantification analysis from single microwell plate (MWP) with multiple samples, nucleic acids and conditions**

A typical relative quantification experiment requiring 3 parameters for a complete analysis is illustrated in the following table, said experiment has 2 target nucleic acids (T1, T2), 2 reference nucleic acids (R1, R2) and 4 samples. The experiment is designed to fit on one 96-well microwell plate (MWP) having for example the assignment of Table 1.

Table 1.1:

| | 0 h | | 1h | | 2h | | 3h | | 4h | | 5h | | | |
|---|---|---|---|---|---|---|---|---|---|---|---|---|---|---|
| | 1 | 2 | 3 | 4 | 5 | 6 | 7 | 8 | 9 | 10 | 11 | 12 | | |
| A | T1 | T2 | T1 | T2 | T1 | T2 | T1 | T2 | T1 | T2 | T1 | T2 | A | Sample 1 |
| B | R1 | R2 | R1 | R2 | R1 | R2 | R1 | R2 | R1 | R2 | R1 | R2 | B | Sample 1 |
| C | T1 | T2 | T1 | T2 | T1 | T2 | T1 | T2 | T1 | T2 | T1 | T2 | C | Sample 2 |
| D | R1 | R2 | R1 | R2 | R1 | R2 | R1 | R2 | R1 | R2 | R1 | R2 | D | Sample 2 |
| E | T1 | T2 | T1 | T2 | T1 | T2 | T1 | T2 | T1 | T2 | T1 | T2 | E | Sample 3 |
| F | R1 | R2 | R1 | R2 | R1 | R2 | R1 | R2 | R1 | R2 | R1 | R2 | F | Sample 3 |
| G | T1 | T2 | T1 | T2 | T1 | T2 | T1 | T2 | T1 | T2 | T1 | T2 | G | Sample 4 |
| H | R1 | R2 | R1 | R2 | R1 | R2 | R1 | R2 | R1 | R2 | R1 | R2 | H | Sample 4 |
| | 1 | 2 | 3 | 4 | 5 | 6 | 7 | 8 | 9 | 10 | 11 | 12 | | |

The experimental condition in this experiment is the point in time the PCR amplification was performed, whereas 6 different time points are considered. The first measurement is performed at time point zero (0h, called base value in the following), the other measurements after 1 hour (1h), 2 hours (2h), 3 hours (3h), 4 hours (4h) and 5 hours (5h).

In the first normalization step relative ratios are calculated separately for each sample and each condition according to the following scheme:

$$\begin{array}{ll} \text{0h:} & E_R^{\mathrm{mean}[Cq\,(R1s_1);\,Cq\,(R2s_1)]}/E_T^{Cq(T1s_1)} \;;\; E_R^{\mathrm{mean}[Cq\,(R1s_1);\,Cq\,(R2s_1)]}/E_T^{Cq(T2s_1)} \\ & E_R^{\mathrm{mean}[Cq\,(R1s_2);\,Cq\,(R2s_2)]}/E_T^{Cq(T1s_2)} \;;\; E_R^{\mathrm{mean}[Cq\,(R1s_1);\,Cq\,(R2s_1)]}/E_T^{Cq(T2s_2)} \\ & E_R^{\mathrm{mean}[Cq\,(R1s_3);\,Cq\,(R2s_3)]}/E_T^{Cq(T1s_3)} \;;\; E_R^{\mathrm{mean}[Cq\,(R1s_1);\,Cq\,(R2s_1)]}/E_T^{Cq(T2s_3)} \\ & E_R^{\mathrm{mean}[Cq\,(R1s_4);\,Cq\,(R2s_4)]}/E_T^{Cq(T1s_4)} \;;\; E_R^{\mathrm{mean}[Cq\,(R1s_1);\,Cq\,(R2s_1)]}/E_T^{Cq(T2s_4)} \end{array}$$

The same calculations are performed for all other experimental conditions (1h - 5h), too.

Consequently, 48 relative ratios are calculated, namely 2 ratios for each of the 4 samples at the 6 points in time. In this example, two reference nucleic acids R1 and R2 are used to calculate the relative ratios, whereby an arithmetic mean value of both reference Cq values is applied.

Afterwards, each measurement is scaled with the respective base value at the base condition 0h in a target-, sample- and condition specific manner providing scaled ratios according to the following scheme:

$$[E_R^{mean[Cq(R1s_1);\,Cq(R2s_1)]}/E_T^{Cq(T1s_1)}]_{ih} / [E_R^{mean[Cq(R1s_1);\,Cq(R2s_1)]}/E_T^{Cq(T1s_1)}]_{0h}$$

$$[E_R^{mean[Cq(R1s_2);\,Cq(R2s_2)]}/E_T^{Cq(T1s_2)}]_{ih} / [E_R^{mean[Cq(R1s_2);\,Cq(R2s_2)]}/E_T^{Cq(T1s_2)}]_{0h}$$

$$[E_R^{mean[Cq(R1s_3);\,Cq(R2s_3)]}/E_T^{Cq(T1s_3)}]_{ih} / [E_R^{mean[Cq(R1s_3);\,Cq(R2s_3)]}/E_T^{Cq(T1s_3)}]_{0h}$$

$$[E_R^{mean[Cq(R1s_4);\,Cq(R2s_4)]}/E_T^{Cq(T1s_4)}]_{ih} / [E_R^{mean[Cq(R1s_4);\,Cq(R2s_4)]}/E_T^{Cq(T1s_4)}]_{0h},$$

with i =1 - 5

The same set of ratios is also calculated for the target nucleic acid T2. Consequently, 40 scaled ratios are calculated, namely 4 ratios for each of the 2 target nucleic acid at 5 points in time. Finally, the analysis of the experiment is performed based on those 40 scale ratios.

**Example 2 : Relative Quantification analysis from multiple MWPs each with multiple samples, nucleic acids and conditions**

In this relative quantification experiment requiring 3 parameters for a complete analysis a setup with two 96-well MWPs is used. Again, the experiment is illustrated in the following tables, said experiment has 2 target nucleic acids (T1, T2), 2 reference nucleic acids (R1, R2) and 4 samples. The experimental condition in this experiment is again the point in time the PCR amplification was performed, whereas 11 different time points are considered. The first measurement is performed at time point zero (0h, called base value), the other measurements after 1 to 10 hours.

Table 2.1:

| | 0 h | | 1h | | 2h | | 3h | | 4h | | 5h | | | |
|---|---|---|---|---|---|---|---|---|---|---|---|---|---|---|
| | 1 | 2 | 3 | 4 | 5 | 6 | 7 | 8 | 9 | 10 | 11 | 12 | | |
| A | T1 | T2 | T1 | T2 | T1 | T2 | T1 | T2 | T1 | T2 | T1 | T2 | A | Sample 1 |
| B | R1 | R2 | R1 | R2 | R1 | R2 | R1 | R2 | R1 | R2 | R1 | R2 | B | Sample 1 |
| C | T1 | T2 | T1 | T2 | T1 | T2 | T1 | T2 | T1 | T2 | T1 | T2 | C | Sample 2 |
| D | R1 | R2 | R1 | R2 | R1 | R2 | R1 | R2 | R1 | R2 | R1 | R2 | D | Sample 2 |
| E | T1 | T2 | T1 | T2 | T1 | T2 | T1 | T2 | T1 | T2 | T1 | T2 | E | Sample 3 |
| F | R1 | R2 | R1 | R2 | R1 | R2 | R1 | R2 | R1 | R2 | R1 | R2 | F | Sample 3 |
| G | T1 | T2 | T1 | T2 | T1 | T2 | T1 | T2 | T1 | T2 | T1 | T2 | G | Sample 4 |
| H | R1 | R2 | R1 | R2 | R1 | R2 | R1 | R2 | R1 | R2 | R1 | R2 | H | Sample 4 |
| | 1 | 2 | 3 | 4 | 5 | 6 | 7 | 8 | 9 | 10 | 11 | 12 | | |

Table 2.2:

| | 0 h | | 6h | | 7h | | 8h | | 9h | | 10h | | | |
|---|---|---|---|---|---|---|---|---|---|---|---|---|---|---|
| | 1 | 2 | 3 | 4 | 5 | 6 | 7 | 8 | 9 | 10 | 11 | 12 | | |
| A | T1 | T2 | T1 | T2 | T1 | T2 | T1 | T2 | T1 | T2 | T1 | T2 | A | Sample 1 |
| B | R1 | R2 | R1 | R2 | R1 | R2 | R1 | R2 | R1 | R2 | R1 | R2 | B | Sample 1 |
| C | T1 | T2 | T1 | T2 | T1 | T2 | T1 | T2 | T1 | T2 | T1 | T2 | C | Sample 2 |
| D | R1 | R2 | R1 | R2 | R1 | R2 | R1 | R2 | R1 | R2 | R1 | R2 | D | Sample 2 |
| E | T1 | T2 | T1 | T2 | T1 | T2 | T1 | T2 | T1 | T2 | T1 | T2 | E | Sample 3 |
| F | R1 | R2 | R1 | R2 | R1 | R2 | R1 | R2 | R1 | R2 | R1 | R2 | F | Sample 3 |
| G | T1 | T2 | T1 | T2 | T1 | T2 | T1 | T2 | T1 | T2 | T1 | T2 | G | Sample 4 |
| H | R1 | R2 | R1 | R2 | R1 | R2 | R1 | R2 | R1 | R2 | R1 | R2 | H | Sample 4 |
| | 1 | 2 | 3 | 4 | 5 | 6 | 7 | 8 | 9 | 10 | 11 | 12 | | |

Based on the amount of different conditions, a set-up with two MWPs was chosen. Alternatively, it is of course possible to arrange all these PCRs on a MWP having a larger amount of well, e.g. a 384 well plate. Here, each MWP has wells for PCRs representing the base condition at time point 0h, such that the second normalization step is performed using base condition amplifications on the same MWP.

The workflow to obtain scaled ratios is the same as outlined in Example 1. Consequently, the first step provides 96 relative ratios and the result of the second step is a set of 80 scaled ratios.

**Example 3 : Relative Quantification analysis from multiple MWPs each with multiple samples, multiple nucleic acids and a run-specific calibrator under different conditions**

In this relative quantification experiment requiring 3 parameters for a complete analysis a set-up with three 96-well MWPs is used, said experiment has 11 target nucleic acids (T1 - T11), 1 reference nucleic acid (R1), 7 samples and 1 calibrator sample. Moreover, there are 3 different experimental conditions, namely untreated samples, samples treated with compound A and samples treated with compound B, whereas the untreated samples provide the base values.

A possible set-up using 3 MWPs is illustrated in the following 3 tables:

Table 3.1:

untreated

| | 1 | 2 | 3 | 4 | 5 | 6 | 7 | 8 | 9 | 10 | 11 | 12 | | |
|---|---|---|---|---|---|---|---|---|---|---|---|---|---|---|
| A | T1 | T2 | T3 | T4 | T5 | T6 | T7 | T8 | T9 | T10 | T11 | R1 | A | Sample 1 |
| B | T1 | T2 | T3 | T4 | T5 | T6 | T7 | T8 | T9 | T10 | T11 | R1 | B | Sample 2 |
| C | T1 | T2 | T3 | T4 | T5 | T6 | T7 | T8 | T9 | T10 | T11 | R1 | C | Sample 3 |
| D | T1 | T2 | T3 | T4 | T5 | T6 | T7 | T8 | T9 | T10 | T11 | R1 | D | Sample 4 |
| E | T1 | T2 | T3 | T4 | T5 | T6 | T7 | T8 | T9 | T10 | T11 | R1 | E | Sample 5 |
| F | T1 | T2 | T3 | T4 | T5 | T6 | T7 | T8 | T9 | T10 | T11 | R1 | F | Sample 6 |
| G | T1 | T2 | T3 | T4 | T5 | T6 | T7 | T8 | T9 | T10 | T11 | R1 | G | Sample 7 |
| H | T1 | T2 | T3 | T4 | T5 | T6 | T7 | T8 | T9 | T10 | T11 | R1 | H | Calibrator |
| | 1 | 2 | 3 | 4 | 5 | 6 | 7 | 8 | 9 | 10 | 11 | 12 | | |

Table 3.2:

treated with Compound A

| | 1 | 2 | 3 | 4 | 5 | 6 | 7 | 8 | 9 | 10 | 11 | 12 | | |
|---|---|---|---|---|---|---|---|---|---|---|---|---|---|---|
| A | T1 | T2 | T3 | T4 | T5 | T6 | T7 | T8 | T9 | T10 | T11 | R1 | A | Sample 1 |
| B | T1 | T2 | T3 | T4 | T5 | T6 | T7 | T8 | T9 | T10 | T11 | R1 | B | Sample 2 |
| C | T1 | T2 | T3 | T4 | T5 | T6 | T7 | T8 | T9 | T10 | T11 | R1 | C | Sample 3 |
| D | T1 | T2 | T3 | T4 | T5 | T6 | T7 | T8 | T9 | T10 | T11 | R1 | D | Sample 4 |
| E | T1 | T2 | T3 | T4 | T5 | T6 | T7 | T8 | T9 | T10 | T11 | R1 | E | Sample 5 |
| F | T1 | T2 | T3 | T4 | T5 | T6 | T7 | T8 | T9 | T10 | T11 | R1 | F | Sample 6 |
| G | T1 | T2 | T3 | T4 | T5 | T6 | T7 | T8 | T9 | T10 | T11 | R1 | G | Sample 7 |
| H | T1 | T2 | T3 | T4 | T5 | T6 | T7 | T8 | T9 | T10 | T11 | R1 | H | Calibrator |
| | 1 | 2 | 3 | 4 | 5 | 6 | 7 | 8 | 9 | 10 | 11 | 12 | | |

Table 3.3:

treated with Compound B

| | 1 | 2 | 3 | 4 | 5 | 6 | 7 | 8 | 9 | 10 | 11 | 12 | | |
|---|---|---|---|---|---|---|---|---|---|---|---|---|---|---|
| A | T1 | T2 | T3 | T4 | T5 | T6 | T7 | T8 | T9 | T10 | T11 | R1 | A | Sample 1 |
| B | T1 | T2 | T3 | T4 | T5 | T6 | T7 | T8 | T9 | T10 | T11 | R1 | B | Sample 2 |
| C | T1 | T2 | T3 | T4 | T5 | T6 | T7 | T8 | T9 | T10 | T11 | R1 | C | Sample 3 |
| D | T1 | T2 | T3 | T4 | T5 | T6 | T7 | T8 | T9 | T10 | T11 | R1 | D | Sample 4 |
| E | T1 | T2 | T3 | T4 | T5 | T6 | T7 | T8 | T9 | T10 | T11 | R1 | E | Sample 5 |
| F | T1 | T2 | T3 | T4 | T5 | T6 | T7 | T8 | T9 | T10 | T11 | R1 | F | Sample 6 |
| G | T1 | T2 | T3 | T4 | T5 | T6 | T7 | T8 | T9 | T10 | T11 | R1 | G | Sample 7 |
| H | T1 | T2 | T3 | T4 | T5 | T6 | T7 | T8 | T9 | T10 | T11 | R1 | H | Calibrator |
| | 1 | 2 | 3 | 4 | 5 | 6 | 7 | 8 | 9 | 10 | 11 | 12 | | |

For each plate the relative ratios are calculated for each target and each sample (incl. the calibrator sample) with respect to the reference nucleic acid R1. Consequently, 88 relative ratios are calculated for each plate.

In a second normalization step the relative ratios are referred to the relative ratios of the calibrator sample providing 77 normalized ratios for each plate.

Finally, in the third normalization step the normalized ratios of the plates for the samples treated with compound A and compound B are referred to the normalized ratios of the plate for the untreated samples providing 154 scaled and normalized ratios.

Finally, the analysis of the experiment is performed based on those 154 scaled and normalized ratios.

## Claims

**1.** A method for the analysis of PCR experiments with a plurality of experimental conditions and a plurality of samples each comprising one or more target nucleic acids and one or more reference nucleic acids, said method comprising

a) a plurality of PCR amplifications of target nucleic acids and reference nucleic acids,
b) a first normalization step based on referring each target nucleic acid amplification to the amplification of one or more reference nucleic acids to obtain relative target/reference ratios,
c) a second normalization step based on referring the relative target/reference ratios calculated in step b) to a relative target/reference ratio obtained at an experimental base condition to obtain scaled target/reference ratios, and
d) an analysis of the PCR experiment based on comparing the scaled target/reference ratios calculated in step c) in a target-, sample- and condition specific manner.

**2.** The method according to claim 1 further comprising a third normalization step based on referring the scaled target/reference ratios calculated in step c) to a scaled target/reference ratio from a calibrator sample to obtain scaled and normalized target/reference ratios, and wherein said analysis of the PCR experiment in step d) is based on comparing said scaled and normalized target/reference ratios in a target-, sample- and condition specific manner.

**3.** The method according to claim 1 further comprising a third normalization step based on referring the relative target/reference ratios calculated in step b) to a relative target/reference ratio from a calibrator sample to obtain normalized target/reference ratios,

wherein the normalization in step c) is based on referring the normalized target/reference ratios to a normalized target/reference ratio obtained at an experimental base condition to obtain scaled and normalized target/reference ratios, and
wherein said analysis of the PCR experiment in step d) is based on comparing said scaled and normalized target/reference ratios in a target-, sample- and condition specific manner.

**4.** The method according to claims 1-3, wherein said experimental conditions are a point in time or a treatment with a certain compound.

**5.** The method according to claims 1-4, wherein all PCR amplifications are performed within wells of one microwell plate.

**6.** The method according to claims 1-4, wherein all PCR amplifications are performed within wells of multiple microwell plates.

**7.** The method according to claims 5-6, wherein said microwell plates are 96 well plates, 384 well plates or 1536 well plates.

**8.** The method according to claims 1-7, wherein the relative target/reference ratios of a sample ($RR_s$) are calculated according to

$$RR_s = E_R^{Cq\,(Rs)}/E_T^{Cq(Ts)}\ ,$$

with:

$E_R$ = PCR efficiency of a reference nucleic acid,
$E_T$ = PCR efficiency of a target nucleic acid and
$C_q$ = cycle of quantification for a reference nucleic acid in a sample (Rs) and for a target nucleic acid in the same sample (Ts).

**9.** The method according to claims 1-8, wherein the scaled target/reference ratios (SR) are calculated according to

$$SR = RR_S / RR_B\ ,$$

with:
$RR_B$ is the relative target/reference ratio in the sample measured at the experimental base condition (B).

**10.** The method according to claims 2-9, wherein the normalized target/reference ratio (NR) are calculated according to

$$NR = RR_S / RR_C\ ,$$

with:

$RR_C$ is the relative target/reference ratio in the calibrator sample.

**11.** The method according to claims 2-10, wherein the scaled and normalized ratios (SNR) are calculated according to

$$SNR = NR_S / NR_B\ ,$$

with:

$NR_S$ the normalized target/reference ratios of a sample at a certain experimental condition and $NR_B$ the normalized target/reference ratio of the same sample at the experimental base condition (B).

**12.** The method according to claims 1-11, wherein the PCR amplification in each well is a mono color amplification.

**13.** The method according to claims 1-11, wherein the PCR amplification in each well is a multi color amplification, preferably a dual color amplification.

# EUROPEAN SEARCH REPORT

Application Number
EP 10 17 7129

| Category | Citation of document with indication, where appropriate, of relevant passages | Relevant to claim | CLASSIFICATION OF THE APPLICATION (IPC) |
|---|---|---|---|
| X | STÅHLBERG A ET AL: "Multiway real-time PCR gene expression profiling in yeast Saccharomyces cerevisiae reveals altered transcriptional response of ADH-genes to glucose stimuli", BMC GENOMICS, BIOMED CENTRAL, LONDON, GB, vol. 9, 16 April 2008 (2008-04-16), pages 1-15, XP002565644, ISSN: 1471-2164, DOI: DOI:10.1186/1471-2164-9-170 * abstract * * page 1 - page 5 * ----- | 1-13 | INV. G06F19/24 C12Q1/68 |
| X | M. KUBISTA, R. SINDELKA: "The Prime technique: Real-time PCR Analysis", G.I.T. LABORATORY JOURNAL, no. 9-10, 2007, pages 33-35, XP007917081, Darmstadt * the whole document * ----- | 1-13 | |
| X | PFAFFL M W: "A new mathematical model for relative quantification in real-time RT-PCR", NUCLEIC ACIDS RESEARCH, OXFORD UNIVERSITY PRESS, GB, vol. 29, no. 9, 1 May 2001 (2001-05-01), pages 2002-2007, XP002607492, ISSN: 1362-4962, DOI: DOI:10.1093/NAR/29.9.E45 * the whole document * ----- | 1-13 | TECHNICAL FIELDS SEARCHED (IPC) G06F C12Q |
| A | M.W. Pfaffl: "Quantification strategies in real-time PCR"; "Chapter 3" In: S.A. Bustin (ed.): "A-Z of quantitative PCR", 2004, La Jolla, CA, USA, XP007917077, pages 87-112, * paragraph [3.2.6] - paragraph [03.5] * ----- -/-- | 1-13 | |

The present search report has been drawn up for all claims

| Place of search | Date of completion of the search | Examiner |
|---|---|---|
| Munich | 10 February 2011 | Kürten, Ivayla |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# EUROPEAN SEARCH REPORT

Application Number
EP 10 17 7129

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| A | US 2003/165832 A1 (SAGNER GREGOR [DE] ET AL) 4 September 2003 (2003-09-04)<br>* abstract *<br>* paragraph [0083] - paragraph [0112] *<br>----- | 1-13 | |
| A | US 6 180 349 B1 (GINZINGER DAVID G [US] ET AL) 30 January 2001 (2001-01-30)<br>* abstract *<br>* column 8, line 50 - column 14, line 7 *<br>* column 21, line 26 - column 28, line 7 *<br>----- | 1-13 | |
| | | | TECHNICAL FIELDS SEARCHED (IPC) |

The present search report has been drawn up for all claims

| Place of search | Date of completion of the search | Examiner |
|---|---|---|
| Munich | 10 February 2011 | Kürten, Ivayla |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

1

EPO FORM 1503 03.82 (P04C01)

## ANNEX TO THE EUROPEAN SEARCH REPORT ON EUROPEAN PATENT APPLICATION NO. EP 10 17 7129

This annex lists the patent family members relating to the patent documents cited in the above-mentioned European search report. The members are as contained in the European Patent Office EDP file on
The European Patent Office is in no way liable for these particulars which are merely given for the purpose of information.

10-02-2011

| Patent document cited in search report | Publication date | Patent family member(s) | Publication date |
|---|---|---|---|
| US 2003165832 A1 | 04-09-2003 | AT 370248 T | 15-09-2007 |
| | | DE 60129869 T2 | 08-05-2008 |
| | | EP 1138783 A2 | 04-10-2001 |
| | | ES 2291238 T3 | 01-03-2008 |
| | | JP 3589638 B2 | 17-11-2004 |
| | | JP 2001314195 A | 13-11-2001 |
| US 6180349 B1 | 30-01-2001 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 1138783 B1
- US 7125691 B